Europäisches Patentamt

European Patent Office

Office européen des brevets

(19)

(11) Veröffentlichungsnummer : **0 119 421**
**B1**

(12) **EUROPÄISCHE PATENTSCHRIFT**

(45) Veröffentlichungstag der Patentschrift :
09.07.86

(51) Int. Cl.⁴ : **C 07 C 39/07**, C 07 C 37/48

(21) Anmeldenummer : 84101092.9

(22) Anmeldetag : 03.02.84

(54) Verfahren zur Isomerisierung und Transalkylierung von alkylierten Phenolen und/oder Phenolderivaten.

(30) Priorität : 11.02.83 DE 3304663

(43) Veröffentlichungstag der Anmeldung :
26.09.84 Patentblatt 84/39

(45) Bekanntmachung des Hinweises auf die Patenterteilung : 09.07.86 Patentblatt 86/28

(84) Benannte Vertragsstaaten :
BE DE FR GB IT NL

(56) Entgegenhaltungen :
DE-A- 1 804 632

(73) Patentinhaber : Union Rheinische Braunkohlen Kraftstoff Aktiengesellschaft
Ludwigshafener Strasse o. Nr. Postfach 8
D-5047 Wesseling (DE)

(72) Erfinder : Keim, Karl-Heinz
Höhenring 31
D-5357 Heimerzheim (DE)
Erfinder : Korff, Joachim, Dr.
Kapellenstrasse 28
D-5303 Bornheim-Merten (DE)

## Beschreibung

Die Erfindung betrifft ein Verfahren zur Isomerisierung und Transalkylierung von alkylierten Phenolen und/oder Phenolderivaten in Gegenwart eines Katalysators, der Eisenoxid enthält und wenigstens ein weiteres Oxid und im Falle des 2,4,6-Trimethylphenols und 2,4-Dimethylphenol von Eisenoxid(en) oder eines Katalysators der Eisenoxid enthält und wenigstens ein weiteres Oxid.

Die Isomerisierung und Transalkylierung von alkylierten Phenolen in Gegenwart saurer, fester Katalysatoren ist seit langem bekannt. So wird in GB-PS 695 464 ein Verfahren beschrieben, bei dem Aluminiumsilikate und Mischoxide von Silicium, Beryllium, Magnesium, Aluminium, Zirkon und Titan zur Transalkylierung und Isomerisierung von Alkylphenolen verwendet werden.

In der DE-OS 1 804 632 wird ein Isomerisierungs- und Transalkylierungsverfahren beschrieben mit Mischkatalysatoren aus Tonerde und Kieselsäure.

Die bekannten Verfahren sind jedoch für den technischen Einsatz nicht zufriedenstellend, da die Umwandlungen zu den z. B. wirtschaftlich interessanten Monomethylphenolen, 2,6- bzw. 2,4-Dimethylphenolen sowie zu 2.3.6-Trimethylphenolen relativ gering sind. Auch bei der Umsetzung höher alkylierter Phenole in Gegenwart relativ großer Mengen von Phenol werden nur unbefriedigende Umsätze erhalten.

Wirksame, technisch brauchbare Katalysatoren, mit denen Alkylphenole mit bis zu 5 Alkylgruppen, auch in Gegenwart von Rückständen aus technischen Anlagen, unter Zusatz relativ geringer Phenolmengen isomerisiert und transalkyliert werden können, sind nicht bekannt, vielmehr wird bei den bekannten Verfahren nur ein geringer Abbau des Rückstandes erzielt.

Es wurde nunmehr überraschend gefunden, daß man mit Katalysatoren, die Eisenoxid und erfindungsgemäß wenigstens ein weiteres Oxid enthalten, bisher unerreicht gute Ergebnisse erzielt und daß man im Falle des 2,4,6-Trimethylphenols und/oder 2,4-Dimethylphenols auch mit Eisenoxid(en) allein sowie mit Eisenoxid und wenigstens einem weiteren Oxid in Gegenwart von Phenol und/oder Alkylphenolen sehr gute Ergebnisse erzielt.

Die Erfindung betrifft daher ein Verfahren zur Isomerisierung und Transalkylierung von alkylierten Phenolen und/oder Phenolderivaten durch Umsetzung der alkylierten Phenole und/oder Phenolderivate gegebenenfalls in Anwesenheit von Phenol bei Temperaturen von 200-550 °C, Drücken von 1-300 bar bei Reak.-Temp. und Verweilzeiten von 0,1-10 h in Gegenwart von Metalloxiden als Katalysator, dadurch gekennzeichnet, daß man die Umsetzung in Gegenwart eines Katalysators durchführt, der

a. wenigstens 60 Gew.% Eisenoxid und

b. wenigstens ein Oxid aus wenigstens einer der folgenden Gruppen :

1. B, Al, Ce
2. Si, Ge, Sn, Ti, Zr
3. Cr, V
4. Li, Na, K, Rb, Cs, Be, Mg, Ca, Sr, Ba, Mn, La

enthält sowie ein Verfahren zur Isomerisierung und Transalkylierung von 2,4,6-Trimethylphenol und/oder 2,4-Dimethylphenol durch Umsetzung derselben in Anwesenheit von Phenol und/oder alkylierten Phenolen bei Temperaturen von 200-550 °C, Drücken von 1-300 bar bei Reaktionstemperatur u. Verweilzeiten von 0,1-10 h in Gegenwart von Metalloxid(en) als Katalysator, dadurch gekennzeichnet, daß man die Umsetzung(en) in Gegenwart eines Katalysators durchführt, der aus Eisenoxid(en) besteht oder eines Katalysators, der wenigstens 60 Gew.% Eisenoxid(e) und wenigstens ein Oxid aus wenigstens einer der folgenden Gruppen :

1. B, Al, Ce, Ga, In, Sc, Y
2. Si, Ge, Sn, Pb, Ti, Zr, Hf
3. Cr, V, Nb, Ta, Mo, W, Re, Co, Ni, Ru, Ir
4. Li, Na, K, Rb, Cs, Be, Mg, Ca, Sr, Ba, Mn, La, Cu, Zn, Cd

enthält.

Bevorzugte Umsetzungsbedingungen sind 250-450 °C, 10-150 bar (Reak.-Temp.) und Verweilzeiten von 0,5-7 Std. Die Umsetzung kann sowohl diskontinuierlich als auch kontinuierlich erfolgen. Besonders geeignete technische Reaktoren sind Röhrenreaktoren, deren Röhren mit dem pelletierten oder granulierten Katalysator gefüllt sind. Der Katalysator kann jedoch auch in stückiger oder anderer Form vorliegen. Auch im Fließbett unter Verwendung eines pulverförmigen Katalysators kann die erfindungsgemäße Umsetzung durchgeführt werden. Dem Fachmann sind diese technischen Apparate und Verfahren bekannt.

Die Untersuchungen haben ergeben, daß es von Vorteil ist, in Wasserstoff-Atmosphäre oder Stickstoff-Atmosphäre zu arbeiten, jedoch auch andere inerte Gase sind geeignet, wie z. B. CO₂ oder Wasserdampf. So kann der Zusatz von Wasser zum Reaktionsgemisch vorteilhaft sein. Bevorzugt wird die Isomerisierung und Transalkylierung der alkylierten Phenole und Phenolderivate in Gegenwart von 5 bis 70 Gew.% Phenol, bezogen auf das Einsatzgemisch, durchgeführt oder auch anderer Phenolderivate, wie z. B. von Kresolen oder von Anisol. Obgleich in der Gasphase gearbeitet werden kann, wird die Arbeitsweise in flüssiger Phase bevorzugt.

Die Aufarbeitung kann durch die bekannten Trennverfahren, wie z. B. Destillation, Extraktion und Kristallisation erfolgen, wobei der bei der Aufarbeitung anfallende Rückstand wieder in den Isomerisierungs- und Transalkylierungsprozeß rückgeführt werden kann, so daß der Rückstand insgesamt weitgehend umgesetzt wird, wobei vorteilhaft Harze vor Rückführung abgetrennt werden.

Anhand der folgenden Tabellen sollen die erfindungsgemäßen Ergebnisse näher erläutert werden.

Die in Tabelle 1 wiedergegebenen Daten wurden mit einem Katalysator der Zusammensetzung

$$Fe_2O_3 : SiO_2 : Cr_2O_3 : BaO = 100 : 2 : 1 : 1$$

erhalten. Die Verweilzeit betrug 1-4 Stunden.

Es wurde unter einem Wasserstoffdruck von 1 bar bzw. 30 bar (bei Raumtemperatur) gearbeitet, bei einer Reaktionstemperatur von 400 °C. Das Einsatzprodukt ist in der 1. Reihe angegeben. Ein Phenolanteil von 20 Gew.% wurde dem Gemisch methylierter Phenole zugesetzt. Der Destillationsrückstand, der bei 190 °C und 3 mbar nicht mehr destillierbar war, beträgt im verwendeten Einsatzprodukt 10,64 Gew.%. Die Summe der Mono- und Dimethylphenole beträgt 2,04 Gew.%. 67,32 Gew.% sind höhere methylierte Phenole. In der 2. Spalte von rechts ist die Summe der bei der Umsetzung erhaltenen wertvollen Mono- und Dimethylphenole enthalten, in der rechten Spalte der Phenolverbrauch bezogen auf eingesetztes Phenol.

Das Einsatzprodukt (ohne zugesetztes Phenol) ist ein technischer Alkylierungsrückstand, aus dem Mono- und Dimethylphenole weitgehend abgetrennt worden sind.

Die Tabelle zeigt, daß mit dem verwendeten Katalysator bei 4 Stunden Verweilzeit ein Produkt erhalten werden kann, das zu 41,62 Gew.% aus Mono- und Dialkylphenolen (27,9 Gew.% Monomethylphenol) besteht, wobei die höheren Alkylphenole um 34,7 % abgenommen haben und der Destillationsrückstand um 40,8 %. Vergleichbare Ergebnisse werden bei Zusatz von Anisol erhalten.

Bei einer Verweilzeit von 1 Stunde und einem Wasserstoffdruck von 30 bar werden bereits 33,63 Gew.% Mono- und Dialkylphenole (23,6 Gew.% Monomethylphenole) erhalten, ebenfalls unter Abbau des Destillationsrückstandes. Auch bei 1 bar Wasserstoffdruck werden 33,71 Gew.% Mono- und Dimethylphenole erhalten.

(Siehe Tabelle 1 Seite 4 f.)

0 119 421

## Tabelle 1

| Katalysator | Vers.-zeit h | Druck bar H$_2$ | % Phenol i.Einsatz | Temp. °C | Produktzusammensetzung in Gew.-% | | | | | | | | | Phenolverbr. bez.a.eig. Phenol |
|---|---|---|---|---|---|---|---|---|---|---|---|---|---|---|
| | | | | | Phenol | o-Kresol | m-Kresol | p-Kresol | 2,4 DMP | 2,6 DMP | höhere Methylphenole | Dest.-Rück. | o-,m-,p-Kresol + 2,4+2,6 DMP | |
| Einsatz | | | | | 2o,o | o,06 | o,ol | o,12 | 1,71 | o,14 | 67,32 | 1o,64 | 2,o4 | – |
| Fe$_2$O$_3$:SiO$_2$ Cr$_2$O$_3$:BaO- 1oo:2:1:1 | 1 | 1 | 2o | 4oo | 7,83 | 11,56 | 7,58 | 4,o7 | 7,22 | 3,28 | 43,36 | 15,1 | 33,71 | 6o,85 |
| " | 1 | 3o | 2o | 4oo | 9,54 | 13,92 | 6,21 | 3,44 | 7,11 | 2,95 | 51,63 | 5,2 | 33,63 | 52,3 |
| " | 2 | 3o | 2o | 4oo | 8,o4 | 13,23 | 8,17 | 4,43 | 8,47 | 4,34 | 47,12 | 6,2 | 38,64 | 59,8 |
| " | 4 | 3o | 2o | 4oo | 8,12 | 13,24 | 9,68 | 5,o | 9,o4 | 4,66 | 43,96 | 6,3 | 41,62 | 59,4 |
| " | 2 | 5o | 2o | 4oo | 8,85 | 14,38 | 6,87 | 3,64 | 7,87 | 3,35 | 52,24 | 2,8 | 36,11 | 55,75 |
| Einsatz Fe$_2$O$_3$:SiO$_2$: Cr$_2$O$_3$:BaO 1oo:2:1:1 | 2 | 5o | % Anisol i.Einsatz 21,2 | 4oo | Anisol 21,2 | o,o5 | o,ol | o,11 | 1,7o | o,13 | 66,87 | 9,93 | 2,o | |
| | 2 | 5o | 21,2 | 4oo | Phenol 8,84 | 14,41 | 7,ol | 3,68 | 7,93 | 3,36 | 52,13 | 2,64 | 36,39 | 55,46 |

0 119 421

Zum Vergleich sind in Tabelle 2 Versuche angegeben, bei denen übliche Katalysatoren verwendet wurden.

Der Versuch, der mit sog. Weißperlen durchgeführt wurde, ist zu vergleichen mit dem Versuch aus Tabelle 1, bei dem bei einer Verweilzeit von 2 Stunden gearbeitet wurde.

Das Ergebnis zeigt, daß die Menge an erhaltenen Mono- und Dimethylphenolen von 38,64 Gew.% (25,83 Monomethylphenole) im Gesamtprodukt auf 30,09 Gew.% (20,2 Gew.% Monomethylphenole) zurückgeht, gleichzeitig der nicht destillierbare Rückstand jedoch auf 25,7 Gew.% ansteigt und 73,25 % des eingesetzten Phenols verbraucht werden.

Auch bei Verwendung von $\gamma$-$Al_2O_3$ wird eine erheblich geringere Ausbeute an Mono- und Dialkylphenolen (18,56 Monomethylphenole) erhalten.

(Siehe Tabelle 2 Seite 6 f.)

5

Tabelle 2

| Katalysator | Vers.-zeit h | Druck bar $H_2$ | % Phenol i.Einsatz | Temp. °C | Produktzusammensetzung in Gew.-% | | | | | | | | % o-,m-,p-Kresol + 2,4+2,6 DMP | % Phenolverl. bcz.a.eig. Phenol |
|---|---|---|---|---|---|---|---|---|---|---|---|---|---|---|
| | | | | | Phenol | o-Kresol | m-Kresol | p-Kresol | 2,4 DMP | 2,6-DMP | höhere Methyl-phenole | Dest.-Rück. | | |
| Weiß-perlen $Al_2O_3$, (1o%) $SiO_2$ (9o%) | 2 | 3o | 2o | 4oo | 5,35 | 7,53 | 9,o9 | 3,59 | 6,78 | 3,1o | 38,86 | 25,7 | 3o,o9 | 73,25 |
| $\gamma$-$Al_2O_3$ | 2 | 3o | 2o | 4oo | 8,62 | 8,56 | 4,83 | 5,17 | 8,14 | 2,54 | 53,34 | 8,8 | 29,24 | 56,9 |

In Tabelle 3 sind Versuche mit verschiedenen erfindungsgemäßen Katalysatoren wiedergegeben und als Vergleichsbeispiel eine Umsetzung mit $Fe_3O_4$ als Katalysator ohne Zusatz eines weiteren Oxids. Der Vergleichsversuch macht die Bedeutung der oxidischen Zusätze deutlich.

(Siehe Tabelle 3 Seite 8 f.)

## Tabelle 3

| Vers. Zeit h | Druck bar $H_2$ | Katalysator | Produktzusammensetzung in Gew.-% | | | | | | | | |
|---|---|---|---|---|---|---|---|---|---|---|---|
| | | | Ph | o-Kresol | m-Kresol | p-Kresol | 2,4-X | 2,6-X | höhere Methylphenole | Dest. Rück. | o-,m-,p-,Kresol 2,4+2,6-X | % Phenolverbr. v. E. |
| -- | -- | - | 2o,o | o,o6 | o,o1 | o,12 | 1,71 | o,14 | 67,32 | 1o,64 | 2,o4 | -- |
| 2 | 3o | $Fe_2O_3:S_iO_2:Cr_2O_3:BaO$ 1oo:2:1:1 | 8,o4 | 13,23 | 8,17 | 4,43 | 8,47 | 4,34 | 47,12 | 6,2 | 38,64 | 59,8 |
| 2 | 3o | $Fe_2O_3:SiO_2:Cr_2O_3$ 1oo:4:1 | 6,79 | 12,51 | 8,16 | 4,33 | 9,16 | 4,68 | 46,57 | 7,8 | 38,84 | 66,o5 |
| 2 | 3o | $Fe_2O_3:SiO_2:MnO:CaO$ 1oo:2:1:o,2 | 7,39 | 13,21 | 7,78 | 4,28 | 8,86 | 4,16 | 49,32 | 5,o | 38,29 | 63,o5 |
| 2 | 3o | $Fe_2O_3:ZrO_2:Cr_2O_3:BaO$ 1oo:2:1:1 | 9,22 | 13,11 | 6,59 | 3,6 | 7,27 | 3,o7 | 5o,44 | 6,7 | 33,64 | 53,9 |
| 2 | 3o | $Fe_2O_3:SiO_2:V_2O_5:BaO$ 1oo:2:1:o,2 | 7,99 | 13,12 | 6,76 | 3,78 | 7,56 | 3,6 | 46,69 | 1o,5 | 34,82 | 6o,o5 |
| 2 | 3o | $Fe_3O_4$ | 16,33 | 7,31 | 3,25 | 2,77 | 6,o7 | 1,54 | 55,33 | 7,4 | 2o,94 | 18,35 |
| 2 | 3o | $Fe_2O_3:SiO_2$ 1oo:2 | 7,5 | 12,42 | 7,51 | 4,44 | 8,32 | 3,97 | 55,84 | 8,2 | 36,66 | 57,1 |

In Tabelle 4 sind unter gleichen Bedingungen wie in Tabelle 3 Versuchsergebnisse mit dem Katalysator der Tabelle 1 bei Einsatz von 2.4.6-Trimethylphenol, unter Zusatz von 22,2 Gew.% Phenol angegeben. Zum Vergleich wurde ein Versuch mit Weißperlen (10 Gew.% $Al_2O_3$, 90 Gew.% $SiO_2$) durchgeführt. In beiden Fällen wurde bei 370 °C gearbeitet.

Ein Beispiel mit 2.4.6-Triethylphenol rein und einem Zusatz von 20 Gew.% Phenol ist ebenfalls in Tabelle 4 angegeben.

(Siehe Tabelle 4 Seite 10 f.)

## Tabelle 4

| Vers.-zeit h | Druck bar $H_2$ | Katalysator | Ph | o-Kresol | m-Kresol | p-Kresol | 2,4-X | 2,6-X | höhere Methylphenole | 2,4,6,-TMP | Dest.-Rück. | o-,m-,p-Kres. 2,4+2,6-X | % Phenolverbi-. v.E. |
|---|---|---|---|---|---|---|---|---|---|---|---|---|---|
| | 2,4,6-TMP 37o°C | | | | | | | | | | | | |
| - | 37o°C | - | 22,2 | o,ol | - | o,o5 | o,61 | o,o2 | o,o2 | 77,1 | - | o,69 | - |
| 2 | 3o | $Fe_2O_3$:$SiO_2$: $Cr_2O_3$:BaO loo:2:1:1 | 2,99 | 13,24 | - | 5,55 | 28,ol | lo,67 | o,35 | 36,89 | 2,3 | 57,47 | 86,41 |
| 2 | 3o | Weißperlen | 2,75 | 6,44 | 5,36 | 3,46 | lo,52 | 4,98 | 27,23 | 25,76 | 13,5 | 3o,76 | 87,5 |

| 2,4,6-Triethyl-phenol rein + 2oGew.% Phenol | Phenol | o-Ethylphenol | m-Ethylphenol | p-Ethylphenol |
|---|---|---|---|---|
| 2 3o $Fe_2O_3$:$SiO_2$: $Cr_2O_3$ loo:2:1:1 | 2,1 | 12,7 | - | 5,3 |

| " | 2,4-Diethylphenol | 2,6-Diethylphenol | höhere Ethylphenole | 2,4,6-Triethylphenol |
|---|---|---|---|---|
| | 29,4 | 9,2 | 7,9 | 33,4 |

Die Untersuchungen der Anmelderin haben überraschend und für den Fachmann unvorhersehbar ergeben, daß bei Einsatz von 2,4,6-Trimethylphenol und 2,4-Dimethylphenol in Gegenwart von Phenol sowohl Eisenoxid(e) selbst als auch Eisenoxid(e) kombiniert mit zahlreichen anderen Metalloxiden zu selektiven Isomerisierungen und Transalkylierungen führen, wobei im Unterschied zum Stand der Technik keine m-methylierten Produkte und nur geringe Mengen an Rückstand entstehen.

Erfindungsgemäße Versuchsergebnisse mit Eisenoxid-Katalysatoren sind in den Tabellen 5 (Einsatzprodukt : 2,4,6-Trimethylphenol) und 6 (Einsatzprodukt : 2,4-Dimethylphenol) wiedergegeben.

Die Versuche wurden bei 400 °C, einem Inertgasdruck von 30 bar (kalt) und einer Verweilzeit von 2 Stunden durchgeführt. Die Untersuchungen der Anmelderin haben ergeben, daß auch bei in den Tabellen nicht wiedergegebenen geänderten Temperaturen, Druck und Verweilzeiten die Selektivität sowie die geringe Rückstandsbildung erhalten bleiben. Der Eisenoxidgehalt in den Katalysatoren lag im allgemeinen bei wenigstens 60 Gew.%. Geringere Eisengehalte führen zu schlechten Ergebnissen. Das Verhältnis von Phenol zu Alkylphenol betrug 50 : 50 Gew.%. Die Mengen in den Tabellen sind in Gewichtsprozent angegeben. Als Vergleichsbeispiel wurde in Tabelle 5 ein sogenannter Weißperlen-Katalysator verwendet, der dem Stand der Technik entspricht. Das Ergebnis zeigt, daß m-Kresol, 2,5-Dimethylphenol, 2,3-Dimethylphenol und 2,3,6-Trimethylphenol neben o-, p-Kresol, 2,4- und 2,6-Dimethylphenol entstehen. Ferner wird eine Rückstandsmenge von 18,6 Gew.% gebildet.

Tabelle 6 enthält ein Vergleichsbeispiel mit Weißperlen als Katalysator und ein Vergleichsbeispiel mit einem eisenoxidfreien Mischkatalysator mit den Komponenten NiO als Hauptkomponente und $SiO_2$, $Cr_2O_3$ und $K_2O$ als weitere Bestandteile.

Auch aus diesen Versuchen geht klar hervor, daß im Unterschied zu den erfindungsgemäßen Versuchsergebnissen weder Selektivität noch geringe Rückstandsbildung erreicht werden.

Das gleiche Ergebnis bezüglich Isomerisierung und Transalkylierung bezüglich 2,4,6-Trimethylphenol und/oder 2,4-Dimethylphenol wird erfindungsgemäß auch dann erhalten, wenn diesen Verbindungen kleinere Mengen anderer Alkylphenole beigemischt sind.

Die in den Tabellen verwendeten Abkürzungen bedeuten :

Ph = Phenol
DMP = Dimethylphenol
TMP = Trimethylphenol
MP = Methylphenole
Kr = Kresole

Aus den Tabellen geht deutlich die Überlegenheit der erfindungsgemäß eingesetzten Katalysatoren hervor.

Die Ergebnisse zeigen, daß die erfindungsgemäß eingesetzten Eisenoxid- bzw. eisenoxidhaltigen Katalysatoren mit den Zusätzen bestimmter Oxide hervorragende Selektivität bei der Umwandlung alkylierter Phenole zu den technisch besonders wichtigen Mono- und Dimethylphenolen ermöglichen, wobei bei Einsatz von 2,4,6-Trimethylphenol und 2,4-Dimethylphenol bisher unerreichte Selektivitäten ohne Bildung m-methylierter Produkte erzielt werden.

## Tabelle 5

### Produktzusammensetzung in Gew.%

| Katalysator | Ph | o-Kresol | m-Kresol | p-Kresol | 2,5-DMP | 2,4-DMP | 2,6-DMP | 2,3-DMP | Σ restl. DMP | 2,4,6-TMP | 2,3,6-TMP | | Dest. Rückst. | 2 o-,p-Kr. 2,4 u. 2,6 | Phenolverbrauch bez. auf Einsatz |
|---|---|---|---|---|---|---|---|---|---|---|---|---|---|---|---|
| Weißperlen $SiO_2/Al_2O_3$ | 11,6 | 13,8 | 15,2 | 6,2 | 9,3 | 8,1 | 4,4 | 3,9 | 4,4 | 2,4 | 2,1 | | 18,6 | 47,7 | 71,0 |
| $Fe_3O_4$ | 29,3 | 19,7 | – | 5,9 | – | 8,0 | 3,1 | – | – | 28,9 | – | | 5,1 | 44,5 | 43,0 |
| $FeO_3/Al_2O_3$ | 24,4 | 21,9 | – | 7,4 | – | 10,1 | 4,2 | – | – | 24,7 | – | | 7,3 | 43,6 | 48,3 |
| $Fe_2O_3/Cr_2O_3/SiO_2/CaO$ | 26,7 | 20,1 | – | 7,8 | – | 15,5 | 5,1 | – | – | 22,4 | – | | 2,4 | 48,5 | 46,6 |
| $Fe_2O_3/MoO_2$ $K_2O$ | 25,3 | 20,3 | – | 6,9 | – | 15,1 | 6,1 | – | – | 26,3 | – | | 1,3 | 48,4 | 47,0 |
| $Fe_2O_3/V_2O_5$ | 26,0 | 18,9 | – | 6,4 | – | 14,7 | 5,4 | – | – | 25,1 | – | | 3,5 | 45,4 | 45,9 |
| $Fe_2O_3/SnO_2/Na_2O$ | 27,4 | 19,7 | – | 7,1 | – | 15,0 | 6,2 | – | – | 23,6 | – | | 1,0 | 48,0 | 44,9 |
| $Fe_2O_3/NiO$ $GeO_2/K_2O$ | 25,9 | 20,1 | – | 7,2 | – | 15,0 | 6,4 | – | – | 24,5 | | | 0,9 | 48,7 | 47,1 |
| $Fe_2O_3/Cr_2O_3$ $SiO_2/BaO$ | 26,3 | 19,9 | – | 8,1 | – | 17,7 | 5,3 | – | – | 24,3 | – | | 0,4 | 49,0 | 46,9 |
| $Fe_2O_3/RuO_2$ | 26,3 | 19,6 | – | 6,8 | – | 14,3 | 5,4 | – | – | 25,2 | – | | 2,4 | 46,1 | 46,2 |
| $Fe_2O_3/MgO$ $SiO_2/BeO$ | 25,5 | 21,7 | – | 6,3 | – | 15,8 | 6,8 | – | – | 23,1 | – | | 1,8 | 49,6 | 47,3 |
| $FeO_3/MnO_2$ $K_2O$ | 26,0 | 20,8 | – | 6,4 | – | 15,1 | 6,5 | – | – | 23,3 | – | | 1,9 | 48,8 | 46,3 |

Tabelle 6
Produktzusammensetzung in Gew.%

2,4

| Katalysator | Ph | o-Kresol | m-Kresol | p-Kresol | 2,5-DMP | 2,4-DMP | 2,6-DMP | 2,3-DMP | Σ restl. DMP | 2,4,6-TMP | 2,3,6-TMP | | Dest. Rückst. | 2 o-,p-Kr. | Phenolver-brauch bez. auf Einsatz |
|---|---|---|---|---|---|---|---|---|---|---|---|---|---|---|---|
| Weißperlen $SiO_2$/$Al_2O_3$ | 12,7 | 13,2 | 17,1 | 5,0 | 8,7 | 6,7 | 2,1 | 2,6 | | 1,1 | 2,7 | | 28,1 | | 69,4 |
| $NiO/SiO_2$ $C_2O_3/K_2O$ | 35,1 | 8,6 | 1,1 | 6,1 | 2,4 | 29,5 | 0,5 | - | - | 1,2 | - | | 15,5 | 15,2 | 40,7 |
| $Fe_2O_3/SiO_2$ | 32,8 | 19,6 | - | 16,9 | - | 25,3 | 2,0 | - | - | 2,7 | - | | 0,7 | 38,5 | 35,3 |
| $Fe_2O_3/V_2O_5$ | 29,1 | 23,7 | - | 18,3 | - | 17,4 | 6,1 | - | - | 3,8 | - | | 2.7 | 48,1 | 40.2 |
| $Fe_2O_3/B_2O_3$ $Cr_2O_3/BeO$ | 35,1 | 15,9 | - | 12,7 | - | 33,1 | 2,1 | - | - | 0,5 | - | | 0,6 | 30,7 | 38,6 |
| $Fe_2O_3/SiO_2$ $Cr_2O_3/K_2O$ | 36,2 | 18,5 | - | 17,6 | - | 21,5 | 3,8 | - | - | 1,9 | - | | 0,5 | 39,9 | 38,3 |
| $Fe_2O_3/SiO_2$ $MoO_2$ | 34,5 | 19,9 | - | 18,7 | - | 18,6 | 3,6 | - | - | 3,1 | - | | 1,6 | 42,2 | 40,3 |
| $Fe_3O_4$ | 32,1 | 17,2 | - | 15,8 | - | 28,0 | 3,1 | - | - | 3,5 | - | | 0,3 | 36,1 | 37,9 |
| $Fe_2O_3/SiO_2$ $MnO_2/CaO$ | 31,2 | 15,7 | - | 16,8 | - | 27,8 | 3,8 | - | - | 2,9 | - | | 1,8 | | 37,6 |
| $Fe_2O_3/PbO$ $Cr_2O_3/BaO$ | 30,0 | 16,7 | - | 16,1 | - | 28,5 | 3,2 | - | - | 3,4 | - | | 2,1 | 36,0 | 43,1 |
| $Fe_2O_3/SiO_2$ $V_2O_5/BaO$ | 36,3 | 17,8 | - | 18,1 | - | 21,1 | 2,7 | - | - | 3,1 | - | | 0,9 | 38,6 | 27,2 |

0 119 421

**0 119 421**

## Patentansprüche

1. Verfahren zur Isomerisierung und Transalkylierung von alkylierten Phenolen und/oder Phenolderivaten durch Umsetzung der alkylierten Phenole und oder Phenolderivate, gegebenenfalls in Anwesenheit von Phenol, bei Temperaturen von 200-550 °C, Drücken von 1-300 bar bei Reaktionstemperatur und Verweilzeiten von 0,1-10 h in Gegenwart von Metalloxiden als Katalysator, dadurch gekennzeichnet, daß man die Umsetzung in Gegenwart eines Katalysators durchführt, der

    a. wenigstens 60 Gew.% Eisenoxid(e) und
    b. wenigstens ein Oxid aus wenigstens einer der folgenden Gruppen :

    1. B, Al, Ce
    2. Si, Ge, Sn, Ti, Zr
    3. Cr, V
    4. Li, Na, K, Rb, Cs, Be, Mg, Ca, Sr, Ba, Mn, La

enthält.

2. Verfahren zur Isomerisierung und Transalkylierung von 2,4,6-Trimethylphenol und/oder 2,4-Dimethylphenol durch Umsetzung derselben in Anwesenheit von Phenol und/oder alkylierten Phenolen bei Temperaturen von 200-550 °C, Drücken von 1-300 bar bei Reaktionstemperatur u. Verweilzeiten von 0,1-10 h in Gegenwart von Metalloxid(en) als Katalysator, dadurch gekennzeichnet, daß man die Umsetzung(en) in Gegenwart eines Katalysators durchführt, der aus Eisenoxid(en) besteht oder eines Katalysators, der wenigstens 60 Gew.% Eisenoxid(e) und wenigstens ein Oxid aus wenigstens einer der folgenden Gruppen :

    1. B, Al, Ce, Ga, In, Sc, Y
    2. Si, Ge, Sn, Pb, Ti, Zr, Hf
    3. Cr, V, Nb, Ta, Mo, W, Re, Co, Ni, Ru, Ir
    4. Li, Na, K, Rb, Cs, Be, Mg, Ca, Sr, Ba, Mn, La, Cu, Zn, Cd

enthält.

3. Verfahren nach den Ansprüchen 1 und 2, dadurch gekennzeichnet, daß man die Umsetzung in Inertgasatmosphäre, insbesondere in Wasserstoff- und/oder Stickstoffatmosphäre durchführt.

4. Verfahren nach den Ansprüchen 1-3, dadurch gekennzeichnet, daß man die Umsetzung in flüssiger Phase durchführt.

5. Verfahren nach den Ansprüchen 1-4, dadurch gekennzeichnet, daß der Temperaturbereich bei 250-450 °C liegt.

6. Verfahren nach den Ansprüchen 1-5, dadurch gekennzeichnet, daß die Verweilzeit bei 0,5-7 Stdn. liegt.

## Claims

1. Process for isomerization and transalkylation of alkylated phenols and/or phenol derivatives by reacting the alkylated phenols and/or phenol derivatives, optionally in the presence of phenol, at temperatures of 200-550 °C, pressures of 1-300 bar at reaction temperature, residence times of 0,1-10 h in the presence of metal oxides as catalysts, characterized in that the reaction is carried out in the presence of a catalyst, which contains :

    a. at least 60 weight-% of iron oxide(s) and
    b. at least one oxide of at least one of the following groups :

    1. B, Al, Ce
    2. Si, Ge, Sn, Ti, Zr
    3. Cr, V
    4. Li, Na, K, Rb, Cs, Be, Mg, Ca, Sr, Ba, Mn, La.

2. Process for isomerization and transalkylation of 2,4,6-trimethylphenol and/or 2,4-dimethylphenol by reacting these alkylphenols in the presence of phenol and/or alkylated phenols at temperatures of 200-550 °C, pressures of 1-300 bar at reaction temperature, and residence times of 0,1-10 h in the presence of metal oxide(s) as catalyst(s), characterized in that the reaction(s) is carried out in the presence of a catalyst, which consists of iron oxide(s) or of a catalyst, which contains at least 60 weight-% of iron oxide(s) and at least one oxide of at least one of the following groups :

    1. B, Al, Ce, Ga, In, Se, Y
    2. Si, Ge, Sn, Pb, Ti, Zr, Hf
    3. Cr, V, Nb, Ta, Mo, W, Re, Co, Ni, Ru, Ir
    4. Li, Na, K, Rb, Cs, Be, Mg, Ca, Sr, Ba, Mn, La, Cu, Zn, Cd.

**0 119 421**

3. Process according to claims 1 and 2, characterized in that the reaction is carried out in an inert atmosphere, preferentially is a hydrogen and/or nitrogen atmosphere.

4. Process according to claims 1-3, characterized in that the reaction is carried out in the liquid phase.

5. Process according to claims 1-4, characterized in that the temperature is from 250-450 °C.

6. Process according to claims 1-5, characterized in that the residence time is 0,5-7 hours.

**Revendications**

1. Procédé pour l'isomérisation et la transalkylation de dérivés de phénols et/ou de phénols alkylés par réaction des dérivés de phénols et/ou phénols alkylés, éventuellement en présence de phénol, à des températures de 200-550 °C, sous les pressions de 1-300 bars à la température de réaction et avec des durées de passage de 0,1-10 h en présence d'oxydes métalliques comme catalyseur, caractérisé en ce que l'on effectue la réaction en présence d'un catalyseur qui contient :

a. de l'oxyde de fer et

b. au moins un oxyde d'au moins un des groupes suivants :

1. B, Al, Ce
2. Si, Ge, Sn, Ti, Zr
3. Cr, V
4. Li, Na, K, Rb, Cs, Be, Mg, Ca, Sr, Ba, Mn, La

2. Procédé pour l'isomérisation et la transalkylation de 2,4,6-triméthylphénol et/ou de 2,4-diméthyl-phénol par réaction de celui-ci en présence de phénol et/ou de phénols alkylés à des températures de 200-550 °C, sous des pressions de 1-300 bars à la température de réaction et pendant des durées de passage de 0,1-10 h en présence d'oxyde(s) métallique(s) comme catalyseur, caractérisé en ce que l'on effectue la (les) réactions(s) en présence d'un catalyseur qui consiste en oxyde(s) de fer ou d'un catalyseur qui contient un (des) oxyde(s) de fer et au moins un oxyde d'au moins un des groupes suivants :

1. B, Al, Ce, Ga, In, Sc, Y
2. Si, Ge, Sn, Pb, Ti, Zr, Hf
3. Cr, V, Nb, Ta, Mo, W, Re, Co, Ni, Ru, Ir
4. Li, Na, K, Rb, Cs, Be, Mg, Ca, Sr, Ba, Mn, La, Cu, Zn, Cd.

3. Procédé selon les revendications 1 et 2, caractérisé en ce que l'on effectue la réaction en atmosphère de gaz inerte, en particulier en atmosphère d'hydrogène et/ou d'azote.

4. Procédé selon les revendications 1-3, caractérisé en ce que l'on effectue la réaction en phase liquide.

5. Procédé selon les revendications 1-4, caractérisé en ce que le domaine de température est de 250-450 °C.

6. Procédé selon les revendications 1-5, caractérisé en ce que la durée de passage est de 0,5-7 h.

15